(19) Europäisches Patentamt
European Patent Office
Office européen des brevets

(11) **EP 2 934 475 B1**

(12) **EUROPEAN PATENT SPECIFICATION**

(45) Date of publication and mention of the grant of the patent:
**06.07.2022 Bulletin 2022/27**

(21) Application number: **13868976.5**

(22) Date of filing: **18.12.2013**

(51) International Patent Classification (IPC):
***A61K 8/58*** *(2006.01)* ***A61Q 17/04*** *(2006.01)*
***A61Q 19/00*** *(2006.01)* ***A61K 8/891*** *(2006.01)*
***A61K 8/892*** *(2006.01)* ***A61K 8/898*** *(2006.01)*
***A61Q 1/02*** *(2006.01)* ***A61Q 19/08*** *(2006.01)*

(52) Cooperative Patent Classification (CPC):
**A61K 8/891; A61K 8/892; A61K 8/898; A61Q 1/02; A61Q 19/08;** A61K 2800/594

(86) International application number:
**PCT/CN2013/089881**

(87) International publication number:
**WO 2014/101703 (03.07.2014 Gazette 2014/27)**

(54) **COSMETIC COMPOSITION**

KOSMETISCHE ZUSAMMENSETZUNG

COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2012 PCT/CN2012/087267**
**01.02.2013 EP 13153664**

(43) Date of publication of application:
**28.10.2015 Bulletin 2015/44**

(73) Proprietors:
- **Unilever Global IP Limited**
**Wirral, Merseyside CH62 4ZD (GB)**
Designated Contracting States:
**CY DE GB IE IT MT**
- **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO RS SE SI SK SM TR**

(72) Inventors:
- **LI, Hangsheng**
**Shanghai 200335 (CN)**
- **MA, Shouwei**
**Shanghai 201210 (CN)**
- **QIU, Qiang**
**Shanghai 200335 (CN)**
- **WANG, Xiuxia**
**Shanghai 200335 (CN)**

(74) Representative: **Fijnvandraat, Arnoldus et al**
**Unilever N.V.**
**Unilever Patent Group**
**Bronland 14**
**6708 WH Wageningen (NL)**

(56) References cited:
**WO-A1-01/91706**
**WO-A1-2005/053634**
**WO-A1-2005/060923**
**WO-A2-03/106614**
**WO-A2-2009/016555**
**WO-A2-2011/148328**
**FR-A1- 2 958 544**
**US-A1- 2007 093 619**
**US-A1- 2009 214 458**
**US-A1- 2010 015 078**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF THE INVENTION

The present invention relates to a cosmetic composition. Particularly, the present invention relates to a cosmetic composition comprising film-forming polymer having a contact angle of at least 85°, non-volatile silicone oil, and cosmetically acceptable carrier, wherein the non-volatile silicone oil comprises dimethiconol, aminosilicone or a mixture thereof and the weight ratio of the film-forming polymer to the non-volatile silicone oil is at least 1:4, wherein the film-forming polymer is selected from silicone resin, non-silicone resin film-forming polymer or a mixture thereof. The composition is useful for topical application to skin, in particular for forming a film with benefits of skin firming, wash-off resistance, abrasion resistance, cumulative deposition of beneficial agent and/or long-lasting deposition of beneficial agent.

### BACKGROUND OF THE INVENTION

Usually, consumers have some skin problems including dryness, wrinkles and fine lines, loose/saggy skin and age spots. Cosmetic composition comprising film-forming polymer may be one solution for consumers to these problems. Film-forming polymer would form a film onto the skin after applying topically and bring immediate firming effect to the skin. Some beneficial agents, for example optical particle and sunscreen agent, may also be delivered onto skin surface together with the film-forming polymer.

There is an increasing interest to develop a skin care composition comprising a film-forming polymer.

US patent application with publication number of US 2008/0233075 A1 disclosed a topical composition comprising a water-soluble film-forming polymer, a bimodal copolymer comprising a first polymeric component with anionic functional groups and a second polymeric component with cationic functional groups, and one or more biological polymers that are derived from a source selected from the group consisting of animals, plants, algae, fungi, and bacteria or are biotechnologically synthesized. Such a topical composition was said to be applied to saggy or wrinkled skin for enhancing the appearance of the skin.

International patent application with publication number of WO 2005/060923 A1 is said to disclose a cosmetic composition comprising a filmogenic system and an emulsifying system. This composition is said to impart to the skin high resistance to water and to sweat and provide prolonged hydration.

However, after applying cosmetic composition, the skin may undergo water washing and abrasion by hand and therefore the film formed by film-forming polymer on the skin may be easily washed away and/or rubbed away and therefore lose the firming benefits. Meanwhile, the beneficial agent would be easily washed off and/or rubbed off and thus can not provide a long-lasting benefit.

Therefore, the present inventors have recognized a need to develop a cosmetic composition with wash-off resistance, abrasion resistance, and/or long-lasting deposition of beneficial agent. Therefore, this invention is directed to a cosmetic composition comprising film-forming polymer having a contact angle of at least 85°, non-volatile silicone oil, and cosmetically acceptable carrier, wherein the non-volatile silicone oil comprises dimethiconol, aminosilicone or a mixture thereof and the weight ratio of the film-forming polymer to the silicone oil is at least 1:4, wherein the film-forming polymer is selected from silicone resin, non-silicone resin film-forming polymer or a mixture thereof. It was surprisingly found that such a cosmetic composition may provide the benefit of skin firming, wash-off resistance, abrasion resistance, cumulative deposition of beneficial agent, and/or long-lasting deposition of beneficial agent.

### DEFINITIONS

Silicone resin

The term "silicone resin" as used herein refers to silicone material which is formed by branched, and/or cage-like oligosiloxanes having three-dimensional structure. Typically, the silicone resin is rigid.

Non-volatile

The term "non-volatile" as used herein means having vapor pressure from 0 to 0.1 mm Hg (13.3 Pa), preferably from 0 to 0.05 mm Hg, more preferably from 0 to 0.01 mm Hg at 25 °C.

Viscosity

Viscosity for the purposes of the present invention means kinematic viscosity at 25°C and is reported as

centiStokes (1 cSt = 1 $mm2.s^{-1}$). Viscosity of fluids such as silicone oil can be determined, for example, by the relevant international standard, such as ISO 3104.

Leave-on and Wash-off

The term "leave-on" as used with reference to compositions herein means a composition that is applied to or rubbed on the skin, and left thereon.

The term "wash-off" as used with reference to compositions herein means a skin cleanser that is applied to or rubbed on the skin and rinsed off substantially immediately subsequent to application.

Refractive Index

Refractive index values referred to herein are those determined at a temperature of 25 °C and a wavelength of 589 nm unless otherwise stated.

Skin

The term "skin" as used herein includes the skin on the face (except eye lids and lips), neck, chest, abdomen, back, arms, hands, and legs. Preferably "skin" means skin on the face.

Film-forming polymer

"Film-forming polymer" as used herein refers to polymer which is capable of forming cohesive and continuous covering over the hair and/or skin when applied to their surface.

Contact angle

Contact angle, as used herein, means the angle at which a water/vapor interface meets a solid surface at a temperature of 25°C. Such an angle may be measured with a goniometer or other water droplet shape analysis systems with water droplet of 5 μl and at 25°C.

Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

**SUMMARY OF THE INVENTION**

In a first aspect, the present invention directed to a cosmetic composition comprising film-forming polymer having a contact angle of at least 85°, non-volatile silicone oil, and cosmetically acceptable carrier, wherein the non-volatile silicone oil comprises dimethiconol, aminosilicone or a mixture thereof and the weight ratio of the film-forming polymer to the non-volatile silicone oil is in the range of 2:5 to 200:1, wherein the film-forming polymer is selected from silicone resin, where contact angle means the angle at which a water/vapor interface meets a solid surface at a temperature of 25 °C where it is measured in accordance with the procedure disclosed in the description, and where silicone resin refers to silicone material which is formed by branched and/or cage-like oligosiloxanes having three-dimensional structure; wherein the cosmetically acceptable carrier is water; and wherein the composition comprises an optical particle.

In a second aspect, the present invention directed to a process for making a cosmetic composition of the first aspect.

All other aspects of the present invention will more readily become apparent upon considering the detailed description and examples which follow.

**DETAILED DESCRIPTION**

The requirement for film-forming polymer is that the film-forming polymer is suitable to be employed in cosmetic composition. For better performance of wash-off resistance, the film-forming polymer preferably has a contact angle of at least 90°, more preferably from 95° to 160°, most preferably from 100° to 120°.

Preferably, the film-forming polymer comprises silicone resin, chitosan, or a mixture thereof. More preferably, the film-forming polymer comprises a silicone resin and most preferably the film-forming polymer is silicone resin.

Preferably, the film-forming polymer is present in the composition in amount of from 0.01 to 20% by weight of the composition, more preferably from 0.2 to 10 %, even more preferably from 0.5 to 7%, and most preferably from 1 to 4% by weight of the composition.

For better performance wash-off resistance, rub resistance, and/or long-lasting deposition of beneficial agent, the weight ratio of the film-forming polymer to the non-volatile silicone oil is at least 2:5. The weight ratio of the film-forming polymer to the non-volatile silicone oil is no more than 200:1, preferably no more than 50:1. The weight ratio of the film-forming polymer to the non-volatile silicone oil is still even more preferably in the range of 4:3 to 40:1 or 1:3 to 2:3, more preferably in the range of 3:2 to 4:1 or 8:1 to 40:1 or 1:3 to 2:3.

The limitation of the silicone resin of the present invention is that the silicone resin is suitable to be used in cosmetic composition and the silicone resin is a film-forming silicone resin. The silicone resin is typically described by the following siloxy monomeric units:

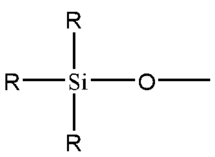

$M=(R)_3SiO_{1/2}$

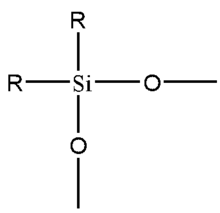

$D=(R)_2SiO_{2/2}$

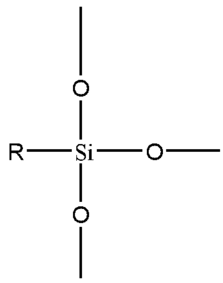

$T=RSiO_{3/2}$

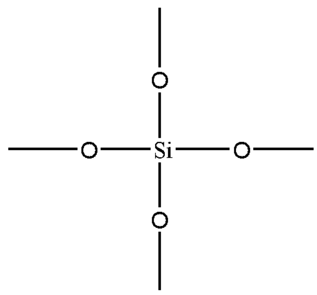

$Q=SiO_{4/2}$

The R group may be selected from saturated or unsaturated hydrocarbon groups. Preferably, the silicone resin of the present invention may be selected from siloxysilicate, silsesquioxane, or a mixture thereof. More preferably, the silicone resin comprises M unit, Q unit, T unit or combination thereof. Even more preferably, the silicone resin comprises MQ silicone resin, T silicone resin, or a mixture thereof.

In some embodiments, the silicone resin preferably comprises MQ silicone resin having the formula of $[(R_1)_3\text{-Si-}O_{1/2}]_a\text{-}(Si\text{-}O_{4/2})_b$, wherein $R_1$ is mutually identical or different, selected from saturated hydrocarbon groups. $R_1$ is preferably selected from $C_1$ to $C_6$ alkyl, and more preferably each $R_1$ is methyl group. Thus, the more preferred MQ silicone resin is trimethylsiloxysilicate. Preferably, a and b independently have values ranging from 10 to 1000, and more preferably from 30 to 200.

In another embodiments, the silicone resin preferably comprises T silicone resin having the formula of $[R_2\text{-Si-}O_{3/2}]_x$, wherein $R_2$ is selected from saturated hydrocarbon groups. $R_2$ is preferably selected from $C_1$ to $C_6$ alkyl, more preferably selected from methyl, ethyl, propyl, butyl, and most preferably propyl. The most preferred T silicone resin is polypropyl silsesquioxane. Preferably, x is less than 2000, more preferably less than 500, but preferably greater than 10, and more preferably greater than 50.

In certain embodiments, the silicone resin preferably comprises a blend of MQ silicone resin and T silicone resin, the weight ratio of MQ silicone resin to T silicone resin is preferably from 1:20 to 20:1 in order to achieve better film-forming performance. More preferably, the weight ratio of MQ silicone resin to T silicone resin is from 1:10 to 10:1, even more preferably from 1:5 to 5:1.

In most preferred embodiments, the composition comprises MQ silicone resin. Preferably the MQ silicone resin is present in amount of at least 20% by weight of the total silicone resin, more preferably at least 40%, even more preferably at least 70%, and still even more preferably at least 85% by weight of the total silicone resin. Most preferably,

MQ silicone resin is present in amount of 100% by weight of the total silicone resin.

Exemplary silicone resin suitable for the present invention includes Dow Corning™ MQ-1640 Flake Resin, a blend of MQ and T Propyl resins, Dow Corning™ MQ-1600 Solid Resin, a 100% active MQ resin, Dow Corning™ 670 Fluid, Cyclopentasiloxane (and) Polypropylsilsesquioxane supplied by Dow Corning.

Preferably, the silicone resin is present in the composition in amount of from 0.01 to 20% by weight of the composition, more preferably from 0.2 to 10 %, even more preferably from 0.5 to 7%, and most preferably from 1 to 4% by weight of the composition.

The non-volatile silicone oil suitable for the present invention comprises dimethiconol, aminosilicone or a mixture thereof. Preferably, the silicone oil comprises aminosilicone. More preferably, the aminosilicone is present in the composition in amount of at least 30% by weight of the total non-volatile silicone, even more preferably at least 60% by weight, and most preferably 100% by weight of the total non-volatile silicone.

Preferably, the non-volatile silicone oil of the present invention is a liquid at 25 °C under atmospheric pressure (760 mmHg). The viscosity of the non-volatile silicone oil is preferably from 20 to 500000 cSt (centi-Stokes) at 25 °C, more preferably from 100 cSt to 50000 cSt, even more preferably from 500 cSt to 15000 cSt, and most preferably from 2000 cSt to 8000 cSt.

The non-volatile silicone oil is preferably present in amount of 0.001 to 15% by weight of the composition, more preferably from 0.01 to 8%, even more preferably from 0.05 to 4%, and most preferably from 0.07 to 2.3% by weight of the composition.

The weight ratio of the silicone resin to the non-volatile silicone oil is at least 1:4. However, for better performance wash-off resistance, rub resistance, and/or long-lasting deposition of beneficial agent, the weight ratio of the silicone resin to the non-volatile silicone oil is preferably at least 1:3, and even more preferably at least 2:5. Preferably, the weight ratio of the silicone resin to the non-volatile silicone oil is no more than 200:1, more preferably no more than 50:1. The weight ratio of the silicone resin to the non-volatile silicone oil is still even more preferably in the range of 4:3 to 40:1 or 1:3 to 2:3, more preferably in the range of 3:2 to 4:1 or 8:1 to 40:1 or 1:3 to 2:3.

Preferably, the dimethiconol has the formula of $HO-[Si(CH_3)_2-O]_a-Si(CH_3)_2-OH$, wherein a is an integer from 10 to 1,200, preferably from 30 to 1000.

Aminosilicone means a silicone containing at least one primary, secondary or tertiary amine group, or a quaternary ammonium group. The primary, secondary, tertiary and/or quaternary amine groups may either form part of the main polymer chain or more preferably be carried by a side or pendant group carried by the polymeric backbone. Suitable aminosilicone for use with the invention are described for example in US 4 185 087.

Aminosilicones suitable for use in the invention will typically have a mole % amine functionality in the range of from 0.1 to 8.0 mole %, preferably from 0.1 to 5.0 mole %, most preferably from 0.1 to 2.0 mole %. In general the amine concentration should not exceed 8.0 mole %.

In a preferred embodiment, the aminosilicone is amodimethicone. Preferably, the amodimethicone has the general formula:

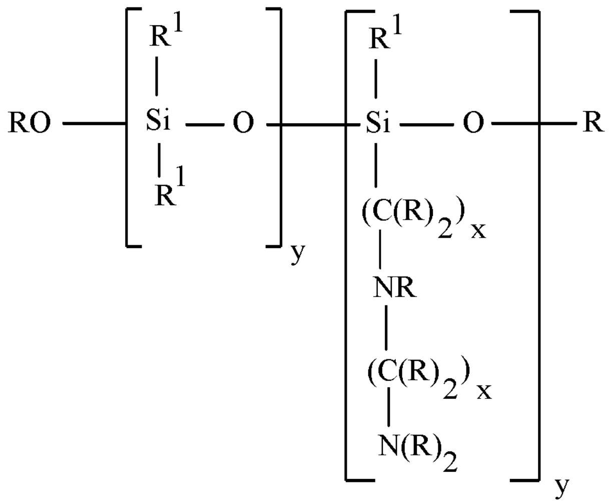

where each R is independently H, or a $C_{1-4}$ alkyl, preferably H; each $R^1$ is independently OR or a $C_{1-4}$ alkyl; and each x is independently an integer from 1 to 4 and each y is greater than zero and independently an integer to yield a polymer having a molecular weight from 500 to 1 million, and preferably from 750 to 25,000, and most preferably from 1,000 to 15,000.

Particularly preferred are silicones comprising an amino glycol copolymer. Especially preferred is Bis (C13-C15 alkoxy) PG amodimethicone such as DC 8500 by Dow Corning.

It is also within the scope of this invention for the aminosilicone to comprise trimethylsilylamodimethicone.

Compositions of the present invention will also include a cosmetically acceptable carrier. Water is the carrier. Amounts of water may, for example, range from 5 to 99%, preferably from 20 to 95%, more preferably from 40 to 90%, optimally between 60 and 85% by weight of the cosmetic composition.

Emollient materials may be included as carriers in compositions of this invention. These may be in the form of silicone oils, synthetic esters and/or hydrocarbons. But the non-volatile silicone oil is different from silicone oil emollient. Amounts of the emollients may range, for example, anywhere from 0.1 to 95%, more preferably between 1 and 50% by weight of the composition.

Silicone oil emollients may be divided into the volatile and nonvolatile variety. Volatile silicone oils are preferably chosen from cyclic (cyclomethicone) or linear polydimethylsiloxanes containing from 3 to 9, preferably from 4 to 5, silicon atoms. In many liquid versions of compositions according to the present invention, the volatile silicone oil emollients may form a relatively large component of the compositions as carriers. Amounts may range, for example, from 5% to 80%, more preferably from 20% to 70% by weight of the composition.

Nonvolatile silicone oil emollients include polyalkyl siloxanes, polyalkylaryl siloxanes and polyether siloxane copolymers. The essentially nonvolatile polyalkyl siloxanes useful herein include, for example, polydimethyl siloxanes with viscosities of from about $5 \times 10^{-6}$ to 0.1 $m^2/s$ at 25 °C. Among the preferred nonvolatile emollients useful in the present compositions are the polydimethyl siloxanes having viscosities from about $1 \times 10^{-5}$ to about $4 \times 10^{-4}$ $m^2/s$ at 25 °C.

Organopolysiloxane crosspolymers can be usefully employed. Representative of these materials are dimethicone/vinyl dimethicone crosspolymers and dimethicone crosspolymers available from a variety of suppliers including Dow Corning (9040, 9041, 9045, 9506 and 9509), General Electric (SFE 839), Shin Etsu (KSG-15, 16 and 18 [dimethicone/phenyl vinyl dimethicone crosspolymer]), and Grant Industries (Gransil brand of materials), and lauryl dimethicone/vinyl dimethicone crosspolymers supplied by Shin Etsu (e.g. KSG-31, KSG-32, KSG-41, KSG-42, KSG-43 and KSG-44). Amounts of the aforementioned silicone elastomers (when present) will usually be from 0.1 to 20% by weight dissolved usually in a volatile silicone oil such as cyclomethicone.

When silicone oil emollients are present in large amounts as carrier and water is also present, the systems may be oil continuous. These normally will require emulsification with a water-in-oil emulsifier such as a dimethicone copolyol (e.g. Abil EM-90 which is cetyl dimethicone copolyol).

Among the ester emollients are:

a) Alkenyl or alkyl esters of fatty acids having 10 to 20 carbon atoms. Examples thereof include isoarachidyl neopentanoate, isodecyl neopentanoate, isononyl isonanoate, cetyl ricinoleate, oleyl myristate, oleyl stearate, and oleyl oleate.

b) Ether-esters such as fatty acid esters of ethoxylated fatty alcohols.

c) Polyhydric alcohol esters. Butylene glycol, ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of $C_1$-$C_{30}$ alcohols. Exemplative is pentaerythrityl tetraethylhexanoate.

d) Wax esters such as beeswax, spermaceti wax and tribehenin wax.

e) Sterols esters, of which cholesterol fatty acid esters are examples thereof.

f) Sugar ester of fatty acids such as sucrose polybehenate and sucrose polycottonseedate.

Of particular use also are the $C_{12-15}$ alkyl benzoate esters sold under the Finsolve brand.

Hydrocarbons which are suitable cosmetically acceptable carriers include petrolatum, mineral oil, $C_{11}$-$C_{13}$ isoparaffins, polyalphaolefins, and especially isohexadecane, available commercially as Permethyl 101A from Presperse Inc.

Humectants of the polyhydric alcohol-type can be employed as cosmetically acceptable carriers. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, glycerol, ethoxylated glycerol, propoxylated glycerol and mixtures thereof. The amount of humectant may range, for example, anywhere from 0.5 to 50%, more preferably between 1 and 15% by weight of the composition. Most preferred is glycerol (also known as glycerin). Amounts of glycerin may range, for example, from 1% to 50%, more preferably from 10 to 35%, optimally from 15 to 30% by weight of the composition.

The cosmetic composition comprises particles which impart opacity to skin, hereafter term "optical particles". Without being bound to any particular theory or explanation, the present inventors believe that optical particles would be embedded into the film by silicone resin and non-volatile silicone oil. Therefore, the optical particles are able to resist water and/or friction and deliver the long-lasting opacity to the skin.

The optical particles are typically particles of high refractive index materials. For example the optical particles may have a refractive index of greater than 1.3, more preferably greater than 1.7 and most preferably from 2.0 to 2.7. Examples of such optical particles are those comprising bismuth oxychloride, boron nitride, barium sulfate, mica, silica, titanium dioxide, zirconium oxide, iron oxide, aluminium oxide, zinc oxide or combinations thereof. Even more preferred are particles comprising zinc oxide, zirconium oxide, titanium dioxide or a combination thereof as these materials have especially high refractive index. Most preferred is titanium dioxide.

For sake of good compatibility with the film-forming polymer and/or non-volatile silicone oil, the optical particle is preferably hydrophobic. More preferably, the optical particle is preferably hydrophobically modified. Even more preferably the optical particle is modified by hydrophobic material selected from fatty acid, silicone oil, wax, and a mixture thereof. The fatty acid preferably comprises oleic acid, stearic acid, or a mixture thereof. The silicone oil may be either the non-volatile silicone oil as mentioned above or any other silicone oil suitable for the modification of optical particle.

Preferably the composition comprises optical particles in an amount of from 0.001 to 10 wt%, more preferably 0.01 to 7 wt%, more preferably still 0.05 to 5 wt% and most preferably 0.1 to 2 wt%. The weight ratio of the film-forming polymer to the optical particle is preferably in the range of from 1:10 to 50:1, more preferably from 1:3 to 10:1, and most preferably from 1:1 to 5:1. The weight ratio of the non-volatile silicone oil to the optical particle is preferably in the range of from 1:50 to 20:1, more preferably from 1:15 to 8:1, and most preferably from 1:5 to 2:1.

When the composition comprises silicone resin and optical particle, the weight ratio of the silicone resin to the optical particle is preferably in the range of from 1:10 to 50:1, more preferably from 1:3 to 10:1, and most preferably from 1:1 to 5:1.

Besides optical particles, the compositions of this invention may include a variety of other functional ingredients. Sunscreen actives may be included in compositions of the present invention. These will be organic compounds having at least one chromophoric group absorbing within the ultraviolet ranging from 290 to 400 nm. Chromophoric organic sunscreen agents may be divided into the following categories (with specific examples) including: p-Aminobenzoic acid, its salts and its derivatives (ethyl, isobutyl, glyceryl esters; p-dimethylaminobenzoic acid); Anthranilates (o-aminobenzoates; methyl, menthyl, phenyl, benzyl, phenylethyl, linalyl, terpinyl, and cyclohexenyl esters); Salicylates (octyl, amyl, phenyl, benzyl, menthyl, glyceryl, and dipropyleneglycol esters); Cinnamic acid derivatives (menthyl and benzyl esters, alpha-phenyl cinnamonitrile; butyl cinnamoyl pyruvate); Dihydroxycinnamic acid derivatives (umbelliferone, methylumbelliferone, methylaceto-umbelliferone); Trihydroxycinnamic acid derivatives (esculetin, methylesculetin, daphnetin, and the glucosides, esculin and daphnin); Hydrocarbons (diphenylbutadiene, stilbene); Dibenzalacetone and benzalacetophenone; Naphtholsulfonates (sodium salts of 2-naphthol-3,6-disulfonic and of 2-naphthol-6,8-disulfonic acids); Dihydroxy-naphthoic acid and its salts; o- and p-Hydroxybiphenyldisulfonates; Coumarin derivatives (7-hydroxy, 7-methyl, 3-phenyl); Diazoles (2-acetyl-3-bromoindazole, phenyl benzoxazole, methyl naphthoxazole, various aryl benzothiazoles); Quinine salts (bisulfate, sulfate, chloride, oleate, and tannate); Quinoline derivatives (8-hydroxyquinoline salts, 2-phenylquinoline); Hydroxy- or methoxy-substituted benzophenones; Uric and vilouric acids; Tannic acid and its derivatives (e.g., hexaethylether); (Butyl carbityl) (6-propyl piperonyl) ether; Hydroquinone; Benzophenones (Oxybenzone, Sulisobenzone, Dioxybenzone, Benzoresorcinol, 2,2',4,4'-Tetrahydroxybenzophenone, 2,2'-Dihydroxy-4,4'-dimethoxybenzophenone, Octabenzone; 4-Isopropyldibenzoylmethane; Butylmethoxydibenzoylmethane; Etocrylene; and 4-isopropyl-dibenzoylmethane). Particularly useful are: 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone, ethyl 4-[bis(hydroxypropyl)]aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl p-dimethylaminobenzoate, 2-phenylbenzimidazole-5-sulfonic acid, 2-(p-dimethylaminophenyl)-5-sulfoniobenzoxazoic acid and mixtures thereof.

Particularly preferred are such materials as ethylhexyl p-methoxycinnamate, available as Parsol MCX®, Avobenzone, available as Parsol 1789®, Dermablock OS® (octylsalicylate) and Mexoryl SX® (with INCI name of Terephthalylidene Dicamphor Sulfonic Acid).

Amounts of the organic sunscreen agent may range, for example, from 0.1 to 15%, more preferably from 0.5% to 10%, optimally from 1% to 8% by weight of the composition.

A variety of thickening agents may be included in the compositions. Illustrative but not limiting are stearic acid, Acrylamide/Sodium Acryloyldimethyltaurate Copolymer (Aristoflex AVC), Hydroxyethyl Acrylate/Sodium Acryloyldimethyltaurate Copolymer, Aluminum Starch Octenyl Succinate, Polyacrylates (such as Carbomers including Carbopol® 980, Carbopol® 1342, Pemulen TR-2® and the Ultrez® thickeners), Polysaccharides (including xanthan gum, guar gum, pectin, carageenan and sclerotium gums), celluloses (including carboxymethyl cellulose, ethyl cellulose, hydroxyethyl cellulose and methyl hydroxymethyl cellulose), minerals (including talc, silica, alumina, mica and clays, the latter being represented by bentonites, hectorites and attapulgites), magnesium aluminum silicate and mixtures thereof. Amounts of the thickeners may range, for example, from 0.05 to 10%, more preferably from 0.3 to 2% by weight of the composition.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, butyl paraben, isobutyl paraben, imidazolidinyl urea, sodium dehydroacetate and benzyl alcohol. The preservatives should be selected having regard for the use of the composition and possible incompatibilities between the preservatives and other ingredients in the composition. Preservatives are preferably employed in amounts ranging from 0.01% to 2% by weight of the composition.

Compositions of the present invention may also contain vitamins and flavonoids. Illustrative water-soluble vitamins are Niacinamide, Vitamin $B_2$, Vitamin $B_6$, Vitamin C and Biotin. Among the useful water-insoluble vitamins are Vitamin A (retinol), Vitamin A Palmitate, ascorbyl tetraisopalmitate, Vitamin E (tocopherol), Vitamin E Acetate and DL-panthenol. A particularly suitable Vitamin $B_6$ derivative is Pyridoxine Palmitate. Among the preferred flavonoids are glucosyl hesperidin and rutin. Total amount of vitamins or flavonoids when present in compositions according to the present invention may range, for example, from 0.001 to 10%, more preferably from 0.01% to 1%, optimally from 0.1 to 0.5% by weight of the composition.

Desquamation agents are further optional components. Illustrative are the alpha-hydroxycarboxylic acids and beta-hydroxycarboxylic acids and salts of these acids. Among the former are salts of glycolic acid, lactic acid and malic acid. Salicylic acid is representative of the beta-hydroxycarboxylic acids. Amounts of these materials when present may range from 0.1 to 15% by weight of the composition.

A variety of herbal extracts may optionally be included in compositions of this invention. Illustrative are pomegranate, white birch (Betula Alba), green tea, chamomile, licorice, boswellia serrata, olive (Olea Europaea) leaf, arnica montana flower, lavandula angustifolia, and extract combinations thereof.

The extracts may either be water soluble or water-insoluble carried in a solvent which respectively is hydrophilic or hydrophobic. Water and ethanol are the preferred extract solvents.

Miscellaneous other adjunct cosmetic ingredients that may be suitable for the present compositions include ceramides (e.g. Ceramide 3 and Ceramide 6), conjugated linoleic acids, colorants (e.g. iron oxides), metal (manganese, copper and/or zinc) gluconates, allantoin, palmitoyl pentapeptide-3, amino acids (e.g. alanine, arginine, glycine, lysine, proline, serine, threonine, glumatic acid and mixtures thereof), trimethylglycine, sodium PCA, chelator like disodium EDTA, opacifiers like titanium dioxide, magnesium aspartate, and combinations thereof. Amounts may, for example, vary from 0.000001 to 3% by weight of the composition.

A small amount of emulsifying surfactant may be present. Surfactants may be anionic, nonionic, cationic, amphoteric and mixtures thereof. Levels may range, for example, from 0.1 to 5%, more preferably from 0.1 to 2%, optimally from 0.1 to 1% by weight. Advantageously the amount of surfactant present should not be sufficient for lather formation. In these instances, less than 2% by weight, preferably less than 1%, and optimally less than 0.5% by weight surfactant is present. Emulsifiers like PEG-100 stearate may be used as well as emulsion stabilizers like cetearyl alcohol and ceteareth-20 may be used and typically in amounts that do not exceed 5 percent by weight of the composition.

Other optional additives suitable for use in the composition of this invention include cationic ammonium compounds to enhance moisturization. Such compounds include salts of hydroxypropyltri ($C_1$-$C_3$ alkyl) ammonium mono-substituted-saccharide, salts of hydroxypropyltri ($C_1$-$C_3$ alkyl) ammonium mono-substituted polyols, dihydroxypropyltri ($C_1$-$C_3$ alkyl) ammonium salts, dihydroxypropyldi ($C_1$-$C_3$ alkyl) mono(hydroxyethyl) ammonium salts, guar hydroxypropyl trimonium salts, 2,3-dihydroxypropyl tri($C_1$-$C_3$ alkyl or hydroxalkyl) ammonium salts or mixtures thereof. In a most preferred embodiment and when desired, the cationic ammonium compound employed in this invention is the quaternary ammonium compound 1,2-dihydroxypropyltrimonium chloride. If used, such compounds typically make up from 0.01 to 30%, and more preferably from about 0.1 to about 15% by weight of the composition.

When cationic ammonium compounds are used, optional additives for use with the same are moisturizing agents such as substituted ureas like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-di-hydroxypropyl urea; N,N,N'-tii-hydroxyethyl urea; tetra(hydroxymethyl) urea; tetra(hydroxyethyl) urea; tetra(hydroxypropyl) urea; N-methyl-N'-hydroxyethyl urea; N-ethyl-N'-hydroxyethyl urea; N-hydroxypropyl-N'-hydroxyethyl urea and N,N'dimethyl-N-hydroxyethyl urea or mixtures thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50% aqueous liquid from AkzoNobel under the trademark Hydrovance. Such substituted ureas, while desirable in moisturizing formulations, are only selected for use when compatible with sunless tanning agent or agents (when used) in the compositions of this invention.

Amounts of substituted urea, when used, in the composition of this invention range from 0.01 to 20%, more preferably from 0.5 to 15%, and most preferably from 2 to 10% based on total weight of the composition and including all ranges subsumed therein.

When cationic ammonium compound and substituted urea are used, in a most especially preferred embodiment at least from 0.01 to 25%, more preferably from 0.2 to 20%, and most preferably from 1 to 15% humectant, like glycerine, is used, based on total weight of the composition and including all ranges subsumed therein.

When making the compositions of this invention, ingredients are typically mixed with moderate shear under atmospheric conditions. When the optical particle is present in the composition it is preferred that the optical particle premixed with the non-volatile silicone oil prior to incorporation into the composition. Therefore, in certain embodiments, the process for making a cosmetic composition preferably comprises the steps of, especially preferably in the case of the optical particle is present and the optical particle is hydrophilic:

a) forming a premix comprising non-volatile silicone oil and optical particle; and
b) combining the premix with film-forming polymer having a contact angle of at least 85° and cosmetically acceptable carrier.

The compositions may be applied topically and preferably 1-4 milligrams of composition is applied per square centimeter of skin. Preferably, the compositions display a pH from 5 to 7. Packaging for the composition of this invention can be a jar or tube as well as any other format typically seen for cosmetic, cream, washing and lotion type products.

It is preferred that the composition is a skin care composition. The composition may be a leave-on composition or a wash-off composition, but preferably a leave-on composition.

The invention also concerns a method for improving skin characteristics comprising the step of topically applying to skin the cosmetic composition of the invention as described. Skin characteristics as used herein often refers to features used to evaluate skin, include but not limit to skin firming, opacity, smoothness, cleanliness, moistening or a combination thereof. Preferably, the skin characteristics comprise skin firming, opacity, or a combination thereof. More preferably skin characteristic is long-lasting opacity. Opacity as used herein often includes masking/reducing blemishes, even skin tone and/or skin lightening

The cosmetic composition of the invention is intended to use for providing any benefit selected from skin firming, wash-off resistance, abrasion resistance, cumulative deposition of beneficial agent, long-lasting beneficial agent deposition, or combination thereof. "Long-lasting" often refers to the beneficial agent (for example optical particle) remains at least 30%, preferably at least 50% after flushing by tap water (25°C) for 1 minute. The beneficial agent preferably comprises optical particle, sunscreen agent or a mixture thereof. More preferably, the beneficial agent is optical particle.

The following examples are provided to facilitate an understanding of the invention. The examples are not intended to limit the scope of the claims.

## EXAMPLES

<u>Wash-off/abrasion resistance test</u>

1. Constructing a calibration curve

The base formulation (sample A in Table 1) was coated evenly onto Bio-skin plate (Color: 30#, ex. BEAULAX, Co. Ltd., Tokyo, Japan) with surface density of 0, 0.5, 1, 1.5, 2, 2.5, 3, 3.5, 4, 5, and 6 mg/$cm^2$. After naturally drying at around 25°C for 8 hours, the lightness of these coated Bio-skin plates was measured using Digieye Imaging System (Verivide, UK). Then the lightness versus surface density was plotted and fitted by a 4 orders' polynomial model to obtain the curve. The R-Square was higher than 0.999 which demonstrated that the polynomial model was suitable to fit the functional relationship between the lightness and the surface density of the base formulation.

2. Wash-off/abrasion experiment

30 mg of samples was coated evenly onto Bio-skin plates with area of 10 $cm^2$. The coated bio-skin was naturally dried at around 25°C for 8 hours. The lightness of the Bio-skin plate (Li) was measured by Digieye Imaging System

(Verivide, UK). The surface density value before wash-off/abrasion experiment ($SD_1$) was obtained according to the calibration curve. The coated bio-skin was soaked into de-ionized water for 30s. Then, a commercial face cleanser (Pond's gold radiance™ Radiance Boosting Cleansing Mousse) with amount of 5 mg/cm$^2$ was applied onto the Bio-skin plate and the coated Bio-skin plate was washed by Martindale abrasion and pilling tester (Type: M235, SDL Altas, USA) with 33.72g of motion plate at the speed of 30 rpm for 1 min. Subsequently, the coated Bio-skin plate was soaked into water for another 1 min and washed by de-ionized water. After naturally drying at around 25°C for 2 hours, the lightness of the Bio-skin plate ($L_2$) was measured again by Digieye Imaging System. The surface density after wash-off/abrasion experiment ($SD_2$) was obtained according to the calibration curve.

The deposition ratio after wash-off/abrasion experiment was calculated by:

$$\text{Deposition ratio} = (SD_2/SD_1) \times 100\%.$$

Measurement of Contact Angle and water soaking test

The contact angles of five film-forming polymers including Dow Corning™ MQ-1640 Flake Resin, Dow Corning™ MQ-1600 Solid Resin, Dow Corning™ 670 Fluid from Dow Corning, Avalure™ UR450 from Lubrizol, Lexorez™ 100 from Inolex were conducted. Dow Corning™ MQ-1600 Solid Resin and Dow Corning™ 670 Fluid were dispersed into dimethicone with a polymer to solvent weight ratio of 1:9. The other three polymers were dispersed into ethanol with a polymer to solvent weight ratio of 1:9.

0.2 ml of film-forming polymer dispersions were dripped evenly onto an ordinary glass sides (about 2 cm×8cm). After the solvents evaporated, uniform films were formed. Drop shape analysis system 100 (DSA 100, Krüss) was used to measure contact angle using deionised water drops of around 5 μL applied to five different points of each film. The contact angle averaged over all 5 drops.

The contact angles of Dow Corning™ MQ-1640 Flake Resin, Dow Corning™ MQ-1600 Solid Resin, Dow Corning™ 670, Avalure™ UR450, and Lexorez™ 100 were 107°, 116°, 114°, 66°, and 28° respectively.

Then, these glass slides coated with polymer films were immersed fully into water for 30 minutes. After that the glass slides were taken out from water and dried. The contact angles of these glass slides were measured again. It was surprisingly found that the contact angles of glass slides coated by Avalure™ UR450, and Lexorez™ 100 were decreased to less than 15°, indicating that the polymer film has been peeled off. In contrast, the contact angle of glass slides coated by Dow Corning™ MQ-1640 Flake Resin, Dow Corning™ MQ-1600 Solid Resin, and Dow Corning™ 670 remained almost the same, manifesting that the polymer films were adhered onto the glass slides firmly.

Example 1

This example demonstrates the inclusion of non-volatile silicone oil into the composition with different film-forming polymer having a contact angle of at least 85° improved the film wash-off resistance, abrasion resistance and therefore deposition of beneficial agent.

A series of cosmetic compositions were formulated as shown in Table 1 below.

The formulations were prepared by the following process. The optical particles were completely dispersed in the oil phase with other ingredients and the silicone resin and/or silicone oil (when present) mixed thoroughly. The resulting oil-based mixture was gradually added to the aqueous phase. The resulting mixture was emulsified under 9,000 rpm of shear stress for 10 minutes at 65 °C and gradually stirred and cooled to room temperature.

The deposition ratios of the samples were measured by following the *Wash-off/abrasion resistance performance test.*

Table 1

| Ingredient (wt%) | Samples | | | | | |
|---|---|---|---|---|---|---|
| | A | B | C | D | 1 | 2 |
| Water | Bal. | Bal. | Bal. | Bal. | Bal. | Bal. |
| Disodium EDTA | 0.10 | 0.10 | 0.10 | 0.10 | 0.10 | 0.10 |
| PEG-100 Stearate (Myij 59 P) | 1.85 | 1.85 | 1.85 | 1.85 | 1.85 | 1.85 |
| Glyceryl stearate | 1.00 | 1.00 | 1.00 | 1.00 | 1.00 | 1.00 |

(continued)

| Ingredient (wt%) | Samples | | | | | |
|---|---|---|---|---|---|---|
| | A | B | C | D | 1 | 2 |
| Caprylic / Capric Triglycerides | 3.00 | 3.00 | 3.00 | 3.00 | 3.00 | 3.00 |
| Aristoflex AVC UL | 1.00 | 1.00 | 1.00 | 1.00 | 1.00 | 1.00 |
| Cyclomethicone/DC 245 | 8.00 | 8.00 | 8.00 | 8.00 | 8.00 | 8.00 |
| Preservative | 0.20 | 0.20 | 0.20 | 0.20 | 0.20 | 0.20 |
| Titanium dioxide [a] | 1.00 | 1.00 | 1.00 | 1.00 | 1.00 | 1.00 |
| Silicone resin-1 [b] | - | 3.00 | - | - | 3.00 | - |
| Silicone resin-2[c] | - | - | 3.00 | - | - | 3.00 |
| Silicone oil-1 [d] | - | - | - | 1.00 | 1.00 | 1.00 |

a. MT700Z, Titanium Dioxide (and) Stearic Acid (and) Aluminium Hydroxide supplied by TAYCA
b. Dow Corning™ MQ-1640 Flake Resin Silicone resin-1 refers to Dow Corning™ MQ-1640 Flake Resin, a blend of MQ and T Propyl resins, supplied by Dow Corning.
c. Dow Corning™ MQ-1600 Solid Resin, a 100% active MQ resin, supplied by Dow Corning.
d. Dow Coming™ 8500 conditioning agent, bis (C13-15 alkoxy) PG amodimethicone, supplied by Dow coming.

As can be seen in Table 2, with the addition of silicone oil into the composition, the deposition ratios after wash-off test for both compositions were increased to above 80%. It was surprisingly found that the inclusion of non-volatile silicone oil of the present invention improved the wash-off resistance, rub resistance, and the long-lasting deposition of optical particles.

In addition, the deposition ratio of composition having MQ silicone resin and non-volatile silicone oil was greater than sum of that of composition having MQ silicone resin alone and that of composition having non-volatile silicone oil alone, showing that there are an unexpected synergic effect between MQ silicone resin and the non-volatile silicone on improving wash-off resistance, rub resistance, and long-lasting deposition of optical particles.

Table 2

| Sample | Silicone resin-1 (wt%) | Silicone resin-2 (wt%) | Silicone oil (wt%) | Deposition Ratio (%) |
|---|---|---|---|---|
| B | 3.00 | 0 | 0 | 59.32 |
| C | 0 | 3.00 | 0 | 36.60 |
| D | 0 | 0 | 1.00 | 30.22 |
| 1 | 3.00 | 0 | 1.00 | 80.20 |
| 2 | 0 | 3.00 | 1.00 | 98.48 |

Example 2

This example demonstrates the effect of weight ratio of silicone resin to silicone oil on the performance of wash-off resistance, abrasion resistance and long-lasting deposition of optical particles.

The formulations in Table 3 were prepared and the deposition ratios of the samples were measured by following similar procedures as described in Example 1.

Table 3

| Ingredient (wt%) | Samples | | | | | | | | | |
|---|---|---|---|---|---|---|---|---|---|---|
| | 3 | 4 | 5 | 6 | 7 | 8 | 9 | 10 | E | F |
| Water | Bal. | Bal. | Bal. | Bal. | Bal. | Bal. | Bal. | Bal. | Bal. | Bal. |
| Disodium EDTA | 0.10 | 0.10 | 0.10 | 0.10 | 0.10 | 0.10 | 0.10 | 0.10 | 0.10 | 0.10 |

(continued)

| Ingredient (wt%) | Samples | | | | | | | | | |
|---|---|---|---|---|---|---|---|---|---|---|
| | 3 | 4 | 5 | 6 | 7 | 8 | 9 | 10 | E | F |
| PEG-100 Stearate (Myij 59 P) | 1.85 | 1.85 | 1.85 | 1.85 | 1.85 | 1.85 | 1.85 | 1.85 | 1.85 | 1.85 |
| Glyceryl stearate | 1.00 | 1.00 | 1.00 | 1.00 | 1.00 | 1.00 | 1.00 | 1.00 | 1.00 | 1.00 |
| Caprylic / Capric Triglycerides | 3.00 | 3.00 | 3.00 | 3.00 | 3.00 | 3.00 | 3.00 | 3.00 | 3.00 | 3.00 |
| Aristoflex AVC UL | 1.00 | 1.00 | 1.00 | 1.00 | 1.00 | 1.00 | 1.00 | 1.00 | 1.00 | 1.00 |
| Cyclomethicone/DC 245 | 8.00 | 8.00 | 8.00 | 8.00 | 8.00 | 8.00 | 8.00 | 8.00 | 8.00 | 8.00 |
| Preservative | 0.20 | 0.20 | 0.20 | 0.20 | 0.20 | 0.20 | 0.20 | 0.20 | 0.20 | 0.20 |
| Titanium dioxide * | 1.00 | 1.00 | 1.00 | 1.00 | 1.00 | 1.00 | 1.00 | 1.00 | 1.00 | 1.00 |
| Silicone resin-2 * | 2.975 | 2.95 | 2.90 | 2.80 | 2.50 | 2.00 | 1.50 | 1.00 | 0.50 | 0.00 |
| Silicone oil-1 * | 0.025 | 0.05 | 0.10 | 0.20 | 0.50 | 1.00 | 1.50 | 2.00 | 2.50 | 3.00 |
| * Same as in Table 1 | | | | | | | | | | |

Table 4 shows the deposition ratios of composition having silicone resin and silicone with different ratios. It was surprisingly found that the compositions of the present invention (samples 3 to 10) had a higher deposition ratio than either sample having silicone resin alone (sample C) or sample having silicone oil alone (Sample F). However, the composition having silicone resin and silicone oil with weight ratio of silicone resin to silicone oil of 1:5 had a deposition ratio lower than both sample having silicone resin alone (sample C) and sample having silicone oil alone (Sample F). Thus, the composition of the present invention can improve wash-off resistance, rub resistance, and long-lasting deposition of optical particles.

Among the compositions of the present invention, generally the compositions having silicone resin and silicone oil with weight ratio of silicone resin to silicone oil greater than 1:5 had higher deposition ratios. More particularly, the compositions having silicone resin and silicone oil with weight ratio of silicone resin to silicone oil of 29:1, 14:1, 2:1, and 1:2 had deposition ratios greater than 65%.

Table 4

| Sample | Silicone resin (wt%) | Silicone oil (wt%) | Weight ratio of silicone resin to silicone oil | Deposition Ratio (%) |
|---|---|---|---|---|
| C | 3.00 | 0 | ∞ | 36.60 |
| 3 | 2.975 | 0.025 | 119:1 | 61.78±0.94 |
| 4 | 2.95 | 0.05 | 59:1 | 61.35±2.20 |
| 5 | 2.90 | 0.10 | 29:1 | 69.90±0.77 |
| 6 | 2.80 | 0.20 | 14:1 | 72.39±0.13 |
| 7 | 2.50 | 0.50 | 5:1 | 56.30±2.20 |
| 8 | 2.00 | 1.00 | 2:1 | 65.58±1.29 |
| 9 | 1.50 | 1.50 | 1:1 | 53.59±0.76 |
| 10 | 1.00 | 2.00 | 1:2 | 85.48±0.55 |
| E | 0.50 | 2.50 | 1:5 | 36.50±0.37 |
| F | 0.00 | 3.00 | 0 | 42.77 |

Example 3

This example demonstrates the effect of addition of other silicone oil on the performance of wash-off resistance, abrasion resistance and long-lasting deposition of optical particles.

The formulations in Table 5 were prepared and the deposition ratios of the samples were measured by following similar procedures as described in Example 1.

Table 5

| Ingredient (wt%) | Samples | | | | |
|---|---|---|---|---|---|
| | G | H | 11 | 12 | 13 |
| Water | Bal. | Bal. | Bal. | Bal. | Bal. |
| Disodium EDTA | 0.10 | 0.10 | 0.10 | 0.10 | 0.10 |
| PEG-100 Stearate (Myij 59 P) | 1.85 | 1.85 | 1.85 | 1.85 | 1.85 |
| Glyceryl stearate | 1.00 | 1.00 | 1.00 | 1.00 | 1.00 |
| Caprylic / Capric Triglycerides | 3.00 | 3.00 | 3.00 | 3.00 | 3.00 |
| Aristoflex AVC UL | 1.00 | 1.00 | 1.00 | 1.00 | 1.00 |
| Cyclomethicone/DC 245 | 8.00 | 8.00 | 8.00 | 8.00 | 8.00 |
| Preservative | 0.20 | 0.20 | 0.20 | 0.20 | 0.20 |
| Titanium dioxide * | 1.00 | 1.00 | 1.00 | 1.00 | 1.00 |
| Silicone resin-1 * | - | - | 1.50 | - | - |
| Silicone resin-2 * | - | - | - | 1.50 | - |
| Silicone resin-3[I] | 3.00 | - | - | - | 1.50 |
| Silicone oil-2 [II] | - | 3.00 | 1.50 | 1.50 | 1.50 |

* Same as in Table 1
I: Dow Corning™ 670 Fluid (45% active), Cyclopentasiloxane (and) Polypropylsilsesquioxane supplied by Dow Corning.
II: Dow Corning™ 1501 (15% active), Cyclopentasiloxane (and) Dimethiconol supplied by Dow Corning.

As can be seen in Table 6, replacing part of silicone resin by less amount of silicone oil, dimethiconol, may at least maintain the deposition ratio of the composition (sample 13) although the silicone oil alone (as in sample E) induced lower deposition ratio of the composition than the silicone resin (as in sample D), or even increased the deposition ratios (sample 11 and 12). The inclusion of silicone oil, dimethiconol improved wash-off resistance, abrasion resistance and long-lasting deposition of optical particles.

Table 6

| Sample | Silicone resin-1 (wt% active) | Silicone resin-2 (wt% active) | Silicone resin-3 (wt% active) | Silicone oil (wt% active) | Deposition Ratio (%) |
|---|---|---|---|---|---|
| B | 3.00 | 0 | 0 | 0 | 59.32 |
| C | 0 | 3.00 | 0 | 0 | 36.60 |
| G | 0 | 0 | 1.35 | 0 | 46.15 |
| H | 0 | 0 | 0 | 0.45 | 32.11 |
| 11 | 1.50 | 0 | 0 | 0.225 | 62.81 |
| 12 | 0 | 1.50 | 0 | 0.225 | 46.62 |
| 13 | 0 | 0 | 0.675 | 0.225 | 59.61 |

Example 4

This example demonstrates the effect of premixing non-volatile silicone oil and optical particle on the performance of wash-off resistance, abrasion resistance and long-lasting deposition of optical particles.

Sample 14 were prepared by following similar procedure as described in Example 1. The procedure for preparing sample 15 was similar with that of sample 14 except that the silicone oil-1 and titanium dioxide were premixed. The deposition ratios of the samples were measured by following similar procedures as described in Example 1.

Table 7

| Ingredient (wt%) | Samples | |
|---|---|---|
| | 14 | 15 |
| Water | Bal. | Bal. |
| Disodium EDTA | 0.10 | 0.10 |
| PEG-100 Stearate (Myij 59 P) | 1.85 | 1.85 |
| Glyceryl stearate | 1.00 | 1.00 |
| Caprylic / Capric Triglycerides | 3.00 | 3.00 |
| Aristoflex AVC UL | 1.00 | 1.00 |
| Cyclomethicone/DC 245 | 8.00 | 8.00 |
| Preservative | 0.20 | 0.20 |
| Titanium dioxide # | 0.90 | 0.90 |
| Silicone resin-1 * | 3.00 | 3.00 |
| Silicone oil-1 * | 0.10 | 0.10 |
| * Same as in Table 1.<br>#: MT700B, Titanium Dioxide without surface coating supplied by TAYCA. | | |

The formation of samples 14 and 15 were same. The difference was that sample 15 was prepared by premixing the silicone oil and optical particle prior to the incorporation into the composition. As can be seen in Table 8, the deposition ratio of sample 15 was higher than that of sample 14. It was demonstrated that forming a premix of silicone oil and optical particle when preparing the composition of the present invention surprisingly enhanced the performance of composition on wash-off resistance, abrasion resistance and long-lasting deposition of optical particles.

Table 8

| Sample | Silicone resin-1 (wt% active) | Silicone oil (wt% active) | Deposition Ratio (%) |
|---|---|---|---|
| 14 | 3.00 | 0.10 | 73.25 |
| 15 | 3.00 | 0.10 | 92.39 |

## Claims

1. A cosmetic composition comprising a film-forming polymer having a contact angle of at least 85°, non-volatile silicone oil, and cosmetically acceptable carrier, wherein the non-volatile silicone oil comprises dimethiconol, aminosilicone or a mixture thereof and the weight ratio of the film-forming polymer to the non-volatile silicone oil is in the range of 2:5 to 200:1 , wherein the film-forming polymer is selected from silicone resin where contact angle means the angle at which a water/vapor interface meets a solid surface at a temperature of 25 °C where it is measured in accordance with the procedure disclosed in the description, and where silicone resin refers to silicone material which is formed by branched and /or cage-like oligosiloxanes having three-dimensional structure;

   wherein the cosmetically acceptable carrier is water;
   ; and
   wherein the composition comprises an optical particle.

2. The composition according to claim 1 wherein the film forming polymer is present in amount of from 0.01 to 20% by weight of the composition, preferably from 0.5 to 7% by weight of the composition.

3. The composition according to any one of the preceding claims wherein the silicone resin comprises MQ silicone resin, T silicone resin, or a mixture thereof, preferably the silicone resin comprises trimethylsiloxy silicate and/or

polypropyl silsesquioxane.

4. The composition according to any one of the preceding claims wherein the silicone resin is present in amount of from 0.01 to 20% by weight of the composition, preferably from 0.5 to 7% by weight of the composition.

5. The composition according to any one of the preceding claims wherein the non-volatile silicone oil comprises aminosilicone, preferably amodimethicone.

6. The composition according to any one of the preceding claims wherein the non-volatile silicone oil is present in amount of 0.01 to 8% by weight of the composition, preferably from 0.07 to 2.3%.

7. The composition according to any one of claims 3 to 6 wherein the weight ratio of silicone resin to the non-volatile silicone oil is in the range of 2:5 to 50:1.

8. The composition according to any one of the preceding claims wherein the optical particle is hydrophobically modified.

9. The composition according to any one of the preceding claims wherein optical particle comprises titanium dioxide, zinc oxide, zirconium oxide, or a mixture thereof.

10. A process for making a cosmetic composition of any one of the preceding claims comprising the step of:

    a) forming a premix comprising non-volatile silicone oil and optical particle; and
    b) combining the premix with film-forming polymer having a contact angle of at least 85°C and cosmetically acceptable carrier,

    wherein the film-forming polymer is selected from silicone resin, non-silicone resin polymer.

**Patentansprüche**

1. Kosmetische Zusammensetzung, umfassend ein filmbildendes Polymer mit einem Kontaktwinkel von mindestens 85°, nicht-flüchtiges Silikonöl und kosmetisch akzeptablen Träger, wobei das nicht-flüchtige Silikonöl Dimethiconol, Aminosilikon oder eine Mischung davon umfasst und das Gewichtsverhältnis des filmbildenden Polymers zu dem nicht-flüchtigen Silikonöl in dem Bereich von 2:5 bis 200:1 liegt, wobei das filmbildende Polymer aus Silikonharz ausgewählt ist, wobei der Kontaktwinkel den Winkel bedeutet, bei dem eine Wasser/Dampf-Grenzfläche auf eine feste Oberfläche bei einer Temperatur von 25°C trifft, wobei er gemäß dem in der Beschreibung dargestellten Verfahren gemessen wird, und wobei sich das Silikonharz auf Silikonmaterial bezieht, welches durch verzweigte und/oder käfigartige Oligosiloxane mit dreidimensionaler Struktur gebildet ist;

    wobei der kosmetisch verträgliche Träger Wasser ist;
    und wobei die Zusammensetzung ein optisches Teilchen umfasst.

2. Zusammensetzung nach Anspruch 1, wobei das filmbildende Polymer in einer Menge von 0,01 bis 20 Gewichts-% der Zusammensetzung, vorzugsweise von 0,5 bis 7 Gewichts-% der Zusammensetzung, vorliegt.

3. Zusammensetzung nach irgendeinem der vorhergehenden Ansprüche, wobei das Silikonharz MQ-Silikonharz, T-Silikonharz oder eine Mischung davon umfasst, wobei das Silikonharz vorzugsweise Trimethylsiloxysilikat und/oder Polypropylsilsesquioxan umfasst.

4. Zusammensetzung nach irgendeinem der vorhergehenden Ansprüche, wobei das Silikonharz in einer Menge von 0,01 bis 20 Gewichts-% der Zusammensetzung, vorzugsweise von 0,5 bis 7 Gewichts-% der Zusammensetzung, vorliegt.

5. Zusammensetzung nach irgendeinem der vorhergehenden Ansprüche, wobei das nicht-flüchtige Silikonöl Aminosilikon, vorzugsweise Amodimethicone, umfasst.

6. Zusammensetzung nach irgendeinem der vorhergehenden Ansprüche, wobei das nicht-flüchtige Silikonöl in einer Menge von 0,01 bis 8 Gewichts-% der Zusammensetzung, vorzugsweise von 0,07 bis 2,3 Gewichts-%, vorliegt.

7. Zusammensetzung nach irgendeinem der Ansprüche 3 bis 6, wobei das Gewichtsverhältnis von Silikonharz zu dem nicht-flüchtigen Silikonöl in dem Bereich von 2:5 bis 50:1 liegt.

8. Zusammensetzung nach irgendeinem der vorhergehenden Ansprüche, wobei das optische Teilchen hydrophob modifiziert ist.

9. Zusammensetzung nach irgendeinem der vorhergehenden Ansprüche, wobei das optische Teilchen Titandioxid, Zinkoxid, Zirconiumoxid oder eine Mischung davon umfasst.

10. Verfahren zur Herstellung einer kosmetischen Zusammensetzung nach irgendeinem der vorhergehenden Ansprüche, umfassend den Schritt von:

a) Bildung einer Vormischung, die nicht-flüchtiges Silikonöl und optisches Teilchen umfasst; und
b) Kombinieren der Vormischung mit filmbildendem Polymer mit einem Kontaktwinkel von mindestens 85° und einem kosmetisch akzeptablen Träger,

wobei das filmbildende Polymer aus Silikonharz, Nichtsilikonharzpolymer ausgewählt ist.

## Revendications

1. Composition cosmétique comprenant un polymère formant un film ayant un angle de contact d'au moins 85°, une huile de silicone non-volatile, et un support cosmétiquement acceptable, où l'huile de silicone non-volatile comprend du diméthiconol, aminosilicone ou un mélange de ceux-ci et le rapport en masse du polymère formant un film à l'huile de silicone non-volatile se trouve dans l'intervalle de 2:5 à 200:1, où le polymère formant un film est choisi parmi une résine de silicone où l'angle de contact indique l'angle auquel une interface eau/vapeur rencontre une surface solide à une température de 25°C où il est mesuré selon la procédure décrite dans la description, et où la résine de silicone fait référence à un matériau de silicone qui est formé par des oligosiloxanes ramifiés et/ou en forme de cage ayant une structure tridimensionnelle ;

dans laquelle le support cosmétiquement acceptable est l'eau ; et
dans laquelle la composition comprend une particule optique.

2. Composition selon la revendication 1, dans laquelle le polymère formant un film est présent dans une quantité de 0,01 à 20 % en masse de la composition, de préférence de 0,5 à 7 % en masse de la composition.

3. Composition selon l'une quelconque des revendications précédentes, dans laquelle la résine de silicone comprend une résine de silicone MQ, résine de silicone T, ou un mélange de celles-ci, la résine de silicone comprend de préférence du triméthylsiloxy silicate et/ou polypropyl silsesquioxane.

4. Composition selon l'une quelconque des revendications précédentes, dans laquelle la résine de silicone est présente dans une quantité de 0,01 à 20 % en masse de la composition, de préférence de 0,5 à 7 % en masse de la composition.

5. Composition selon l'une quelconque des revendications précédentes, dans laquelle l'huile de silicone non-volatile comprend un aminosilicone, de préférence amodiméthicone.

6. Composition selon l'une quelconque des revendications précédentes, dans laquelle l'huile de silicone non-volatile est présente dans une quantité de 0,01 à 8 % en masse de la composition, de préférence de 0,07 à 2,3 %.

7. Composition selon l'une quelconque des revendications 3 à 6, dans laquelle le rapport en masse de résine de silicone à l'huile de silicone non-volatile se trouve dans l'intervalle de 2:5 à 50:1.

8. Composition selon l'une quelconque des revendications précédentes, dans laquelle la particule optique est hydrophobiquement modifiée.

9. Composition selon l'une quelconque des revendications précédentes, dans laquelle la particule optique comprend du dioxyde de titane, oxyde de zinc, oxyde de zirconium, ou un mélange de ceux-ci.

**10.** Procédé pour la fabrication d'une composition cosmétique selon l'une quelconque des revendications précédentes comprenant les étapes de :

a) formation d'un prémélange comprenant une huile de silicone non-volatile et une particule optique ; et
b) combinaison du prémélange avec un polymère formant un film ayant un angle de contact d'au moins 85°C et un support cosmétiquement acceptable,

dans lequel le polymère formant un film est choisi parmi un polymère de résine de silicone, résine de non-silicone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080233075 A1
- WO 2005060923 A1
- US 4185087 A